# EUROPEAN PATENT SPECIFICATION

(11) **EP 4 538 713 B1**
(45) Date of publication and mention of the grant of the patent: **11.11.2026**
(21) Application number: 25156513.1
(22) Date of filing: 14.01.2020
(51) Int. Cl.: G01N 31/22, G01N 33/24, G01N 33/84

(54) **SOIL ANALYIS COMPOSITIONS AND METHODS**
BODENANALYTISCHE ZUSAMMENSETZUNGEN UND VERFAHREN
COMPOSITIONS D'ANALYIS DE SOL ET PROCÉDÉS ASSOCIÉS

(30) Priority: 15.01.2019 US 201962792542 P
(43) Date of publication of application: 16.04.2025
(62) Document number(s) of the earlier application(s) in accordance with Art. 76 EPC: 20702381.3 / 3 911 948
(73) Proprietor: Precision Planting LLC, Tremont, IL 61568 (US)
(72) Inventor: PETROSKI, Richard, Tremont, 61568 (US); NELSON, Rachel, Tremont, 61568 (US)
(74) Representative: AGCO Intellectual Property Department

(56) References cited:
- EP-A2- 0 404 590
- JP-A- S 533 391
- US-A- 3 853 469

## Description

### CROSS-REFERENCE TO RELATED APPLICATIONS

This application is a PCT International Application claiming the benefit of United States Provisional Patent Application No. 62/792542, filed on January 15, 2019.

### BACKGROUND

Soil analysis of agricultural fields allows a grower to know whether there are sufficient amounts of nutrients in the soil for planting. If one or more nutrients is deficient, then the nutrient can be added to soil.

Typically, soil samples are obtained via a bag and tag system in which soil samples are obtained from multiple locations in a field, placed in a bag, tagged with the information of where the soil sample was taken in the field, and then shipped to a lab for testing. This takes time, and the grower needs to wait for the test results before being able to take action.

Current soil tests are usually laborious in that soil samples are first dried to a target moisture and then ground to provide a dry soil of uniform particle size for testing. This adds processing time and costs to the testing procedure.

There are many standardized soil tests available today, such as measurement of pH with a pH meter and measurement of soil nutrients by atomic spectroscopy. These tests, however, were designed for laboratory testing, and they are not suitable for an on the go soil sampling system.

It would be desirable to test soil samples on the go with soil tests that can provide results while in the field.

### BRIEF SUMMARY

The present invention relates to a composition comprising: a soil extract, a first solution comprising a surfactant that forms a turbid solution in the presence of molybdate and phosphorous; and a second solution comprising a molybdate salt in a 5 to 7 N acid.

In one embodiment, a method of measuring phosphorous amount of a soil slurry comprising: a) obtaining a soil extract; b) adding a surfactant that forms a turbid solution in the presence of molybdate and phosphorous, a molybdate salt, and an acid to the soil extract to form a mixture; and c) measuring absorbance of the mixture.

The present invention is expressly not limited to use with soil sampling at any particular location but can be used at any location.

### DETAILED DESCRIPTION

The features and benefits of the invention are illustrated and described herein by reference to exemplary ("example") embodiments. This description of exemplary embodiments is intended to be read in connection with the accompanying drawings, which are to be considered part of the entire written description. Accordingly, the disclosure expressly should not be limited to such exemplary embodiments illustrating some possible non-limiting combination of features that may exist alone or in other combinations of features.

As used throughout, any ranges disclosed herein are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

The compositions and methods described below can be used with on the go soil sampling systems, such as those described in U.S. Patent Publication No. US2018/0124992, or in U.S. Application Nos. 62/696,271, filed 10 July 2018; 62/729,623, filed 11 September 2018; and 62/745,606, filed 15 October 2018. Also, the tests and methods can be used in a laboratory. When used in on the go systems, it is desirable to obtain results in a short period of time (shorter than traditional laboratory testing) so that multiple samples can be tested while traversing the field. This allows a grower to adjust application rates of nutrients in real time.

In one embodiment, the compositions and methods below can be used in a colorimetric analysis system. This allows for a minimization of testing equipment.

According to the present invention phosphorus is tested using a combination of reagent including a surfactant, such as Triton X-100 surfactant (Polyethylene glycol *p*-(1,1,3,3-tetramethylbutyl)-phenyl ether, CAS 9002-93-1), and a molybdate salt, such as ammonium molybdate tetrahydrate, in an acid solution. Acids that can be used include, but are not limited to sulfuric acid, nitric acid, and hydrochloric acid. In one embodiment, an amount of Triton X-100 is 0.5 to 2 weight % of the composition. In one embodiment, the acid has a concentration of 5 to 7 N, optionally 6N. In one embodiment, the composition includes ammonium molybdate tetrahydrate in a range of 15 to 25 mM, optionally 20.2 mM, in a 5 to 7 N, optionally 6N, sulfuric acid solution. In other embodiments, sodium molybdate can be used in place of ammonium molybdate in the same molar amounts. The surfactant is one that forms a turbid composition in the presence of molybdate and phosphorous.

Optionally, a flocculating agent can be added (as described above, for example using polyacrylamide as described above, such as at 0.01 weight %).

The reagent can be added to the soil solution in a weight ratio of soil:Triton X-100:ammonium molybdate tetrahydrate of 1:26:3. After mixing, the solution will become turbid. In one embodiment, absorbance can be read after 10 seconds of mixing. Absorbance can be read via a spectrophotometer at 510 nm. In one embodiment, the absorbance machine is an Agilent Cary 100 UV-Vis spectrophotometer.

Soil samples are prepared as for typical laboratory testing by drying, crushing, and filtering to less than 2 mm particle size. Multiple samples are prepared to provide a sufficient number to generate a calibration curve. The traditional test for phosphorus in soil is conducted using the Bray-1 or the Bray-2 extractants, and then measuring by ICP-OES. The test can be referenced in Recommended Chemical Soil Test Procedures for the North Central Region, North Central Regional Research Publication No. 221, Missouri Agricultural Experiment Station SB 1001, August 2015 (http://extension.missouri.edu/explorepdf/specialb/sb1001.pdf). The samples are also measured using the above phosphorus test and the results are correlated for a correlation curve.

A calibration curve can be obtained and used with the correlation chart. Soil slurry before adding indicator composition is measured for absorbance to establish a blank reading. The indicator composition (as described above) is then added to this sample and remeasured for absorbance. The difference in the absorbance readings is used for a calibration curve. This calibration can be done as needed, such as once per day.

### Cartridge

In one embodiment, a multichamber cartridge can be provided in which each chamber contains one of the above compositions in a combination that tests for at least two of the above listed tests (e.g., two or more of the pH test, buffer pH test, potassium test, phosphorous test, calcium test, and/or magnesium tests). In one embodiment, the cartridge has a chamber for the pH test composition, a chamber for the buffer pH test composition, a chamber for the potassium test composition, a chamber for the phosphorous test composition, a chamber for the calcium test composition, and a chamber for the magnesium test composition. In one embodiment, any of the cartridges can contain an additional chamber that does not contain any of the compositions for the above tests.

### EXAMPLES

### Example 1 - Phosphorus Analysis

An experiment was performed to test phosphorus analysis of soil. Water used in analysis is type 1 water. The experiment of this example uses the following components:
Solution A 1 % weight/volume Triton X-100; 1 gram per 100 mL water
Solution B Ammonium molybdate; 2.5 grams of ammonium molybdate tetrahydrate is dissolved in 100 mL of 6N sulfuric acid (60 mL of 10 N sulfuric acid is added to 40 mL of water).

The preparation of standards used soil test results to get the amount of phosphorus in soils. This is a range of 1 to 50 µg per gram of soil for Bray 1 extract (weak Bray) and a range of 10 to 150 for Bray 2 (strong Bray). Using 100 mg of soil the amount of phosphorus is one tenth of the above values in the soil sample. For example, 50 µg per gram P in soil gives 5 µg in the 100 mg soil sample. Use 1 mL of Bray 1 for routine analysis, to measure the amount of P available to the plant and 1 mL of Bray 2, to estimate the stored P (in the "bank" for the plant when made available by microbes in the soil). A 200 µL aliquot of the soil extract would have on fifth of the total extracted phosphate.

The standard preparation further used a stand stock solution of 6 µg per mL to prepare standards. Aliquots would be 0, 50, 100 and 200 µL for 0, 0.3, 0.6 and 1.2 µg of phosphorus. Bring a/l standard volumes to 200 µL total with water.

The standard preparation further used 200 µL of soil extract for Bray 1 analysis and 100 µL of soil extract for Bray 2 analysis. The dilution factor for Bray 1 analysis is 50-1 and the dilution factor for Bray 2 analysis is 100-1.

The standard preparation further used samples, blanks and samples should have the same amount of Bray 1 or Bray 2 extractant (200 µL for Bray 1 and 100 µL for Bray 2), then added water to bring the total volume to 2.7 mL.

The experiment included the following methodology: added 30 µL of Reagent A, 1 % Triton X-100, to the samples blank and standards and then mixed and wait 2 minutes. Subsequently, added 300 µL of Reagent B, 2.5% Ammonium molybdate in 6 N sulfuric acid. Mix and wait up to 20 min for the turbidity to develop. The absorbances at 510 nm were then read, using the CONCENTRATION feature of the Cary 100 instrument. The amount of total phosphorus in the soil is the concentration times the dilution factor used.

## Claims

1. A composition comprising:
a. a soil extract;
b. a first solution comprising a surfactant that forms a turbid solution in the presence of molybdate and phosphorous; and
c. a second solution comprising a molybdate salt in a 5 to 7 N acid.

2. The composition according to claim 1, wherein the molybdate salt is ammonium molybdate tetrahydrate, and the acid is sulfuric acid.

3. The composition according to claim 2, wherein the ammonium molybdate tetrahydrate is present in a range of 15 to 25 mM, in the 5 to 7 N sulfuric acid solution.

4. The composition according to any one of claims 1 to 3, wherein the surfactant is polyethylene glycol *p*-(1,1,3,3-tetramethylbutyl)-phenyl ether.

5. The composition according to claim 4, wherein the polyethylene glycol *p*-(1,1,3,3-tetramethylbutyl)-phenyl ether is present in an amount of 0.5 to 2 weight % of the composition.

6. A method of measuring phosphorous amount of a soil slurry comprising:
a. obtaining a soil extract;
b. adding a surfactant that forms a turbid solution in the presence of molybdate and phosphorous, a molybdate salt, and an acid to the soil extract to form a mixture; and
c. measuring absorbance of the mixture.

7. The method according to claim 6, wherein the molybdate salt is ammonium molybdate tetrahydrate, and the acid is sulfuric acid.

8. The method according to claim 7, wherein the molybdate salt is provided as a composition that includes ammonium molybdate tetrahydrate in a range of 15 to 25 mM in a 5 to 7 N sulfuric acid solution.

9. The method according to any one of claims 6 to 8, wherein the surfactant is polyethylene glycol *p*-(1,1,3,3-tetramethylbutyl)-phenyl ether.

10. The method according to claim 9, wherein the reagents are added to the soil solution in a weight ratio of soil: polyethylene glycol *p*-(1,1,3,3-tetramethylbutyl)-phenyl ether:ammonium molybdate tetrahydrate of 1:26:3.

## Patentansprüche

1. Zusammensetzung, umfassend:
a. einen Bodenextrakt;
b. eine erste Lösung, die ein Tensid umfasst, das in Gegenwart von Molybdat und Phosphor eine trübe Lösung bildet; und
c. eine zweite Lösung, die ein Molybdatsalz in einer 5 bis 7 N Säure umfasst.

2. Zusammensetzung nach Anspruch 1, wobei es sich bei dem Molybdatsalz um Ammoniummolybdat-Tetrahydrat und bei der Säure um Schwefelsäure handelt.

3. Zusammensetzung nach Anspruch 2, wobei das Ammoniummolybdat-Tetrahydrat in einem Bereich von 15 bis 25 mM in der 5 bis 7 N Schwefelsäurelösung vorliegt.

4. Zusammensetzung nach einem der Ansprüche 1 bis 3, wobei es sich bei dem Tensid um Polyethylenglykol-*p-*(1,1,3,3-tetramethylbutyl)phenylether handelt.

5. Zusammensetzung nach Anspruch 4, wobei der Polyethylenglykol-*p*-(1,1,3,3-tetramethylbutyl)phenylether in einer Menge von 0,5 bis 2 Gew.-% der Zusammensetzung vorliegt.

6. Verfahren zum Messen der Phosphormenge einer Bodenaufschlämmung, umfassend:
a. Gewinnen eines Bodenextrakts;
b. Zugeben eines Tensids, das in Gegenwart von Molybdat und Phosphor eine trübe Lösung bildet, eines Molybdatsalzes und einer Säure zum Bodenextrakt unter Bildung eines Gemischs; und
c. Messen der Extinktion des Gemischs.

7. Verfahren nach Anspruch 6, wobei es sich bei dem Molybdatsalz um Ammoniummolybdat-Tetrahydrat und bei der Säure um Schwefelsäure handelt.

8. Verfahren nach Anspruch 7, wobei das Molybdatsalz als Zusammensetzung bereitgestellt wird, die Ammoniummolybdat-Tetrahydrat in einem Bereich von 15 bis 25 mM in einer 5 bis 7 N Schwefelsäurelösung enthält.

9. Verfahren nach einem der Ansprüche 6 bis 8, wobei es sich bei dem Tensid um Polyethylenglykol-*p*-(1,1,3,3-tetramethylbutyl)phenylether handelt.

10. Verfahren nach Anspruch 9, wobei die Reagenzien der Bodenlösung in einem Gewichtsverhältnis von Boden zu Polyethylenglykol-*p*-(1,1,3,3-tetramethylbutyl)phenylether zu Ammoniummolybdat-Tetrahydrat von 1:26:3 zugesetzt werden.

## Revendications

1. Composition comprenant :
a. un extrait de sol ;
b. une première solution comprenant un tensioactif qui forme une solution trouble en présence de molybdate et de phosphore ; et
c. une deuxième solution comprenant un sel de molybdate dans un acide 5 à 7 N.

2. Composition selon la revendication 1, dans laquelle le sel de molybdate est le tétrahydrate de molybdate d'ammonium, et l'acide est l'acide sulfurique.

3. Composition selon la revendication 2, dans laquelle le tétrahydrate de molybdate d'ammonium est présent dans une plage de 15 à 25 mM, dans la solution d'acide sulfurique 5 à 7 N.

4. Composition selon l'une quelconque des revendications 1 à 3, dans laquelle le tensioactif est l'éther de p-(1,1,3,3-tétraméthylbutyl)-phényle de polyéthylèneglycol.

5. Composition selon la revendication 4, dans laquelle l'éther de p-(1,1,3,3-tétraméthylbutyl)-phényle de polyéthylèneglycol est présent en une quantité de 0,5 à 2 % en poids de la composition.

6. Procédé de mesure de la quantité de phosphore d'une boue de sol comprenant :
a. obtenir un extrait de sol ;
b. ajouter un tensioactif qui forme une solution trouble en présence de molybdate et de phosphore, un sel de molybdate et un acide à l'extrait de sol pour former un mélange ; et
c. mesurer l'absorbance du mélange.

7. Procédé selon la revendication 6, dans lequel le sel de molybdate est le tétrahydrate de molybdate d'ammonium, et l'acide est l'acide sulfurique.

8. Procédé selon la revendication 7, dans lequel le sel de molybdate est fourni sous forme d'une composition qui comprend du tétrahydrate de molybdate d'ammonium dans une plage de 15 à 25 mM dans une solution d'acide sulfurique 5 à 7 N.

9. Procédé selon l'une quelconque des revendications 6 à 8, dans lequel le tensioactif est l'éther de p-(1,1,3,3-tétraméthylbutyl)-phényle de polyéthylèneglycol.

10. Procédé selon la revendication 9, dans lequel les réactifs sont ajoutés à la solution de sol dans un rapport pondéral sol:éther de p-(1,1,3,3-tétraméthylbutyl)-phényle de polyéthylèneglycol:tétrahydrate de molybdate d'ammonium de 1:26:3.
